# EUROPEAN PATENT APPLICATION

(11) **EP 2 959 929 A1**
(43) Date of publication of application: **30.12.2015**
(21) Application number: 14306007.7
(22) Date of filing: 26.06.2014
(51) Int. Cl.: A61M 5/14, A61B 19/02

(54) **Holding device for holding lines or cables of medical devices**

(71) Applicant: Fresenius Vial SAS, 38590 Brézins (FR)
(72) Inventor: Couillaud, Frederic, 38500 Coublevie (FR)

(57) **Abstract**

A holding device (4) for holding lines (20) or cables of medical devices (2), the holding device (4) comprising a holder (41) having a multiplicity of attachment elements (42-45) integrally connected to each other for receiving a multiplicity of lines (20) or cables, each attachment element (42-45) comprising a guide opening (423-453) for receiving one or several line (20) or cable and a tab section (420-450) extending along a circumferential direction about a longitudinal direction (L) around the guide opening (423-453). Herein, the tab section (420-450) of a first of the multiplicity of attachment elements (42-45) is positioned at a different height, when viewed along the longitudinal direction (L), than the tab section (420-450) of a second of the multiplicity of attachment elements (42-45). In this way a holding device is provided which in an easy and comfortable manner allows for arranging a multiplicity of infusion lines for example on a rack carrying a multiplicity of medical devices, in particular infusion pumps.

## Description

The invention relates to a holding device for holding lines or cables of medical devices according to the preamble of claim 1.

A holding device of this kind comprises a holder having a multiplicity of attachment elements integrally connected to each other for receiving a multiplicity of lines or cables. Each attachment element herein comprises a guide opening for receiving one or several line or cable and a tab section extending along a circumferential direction about a longitudinal direction around the guide opening.

A holding device known from US 2012/0277682 A1 is constituted to hold and removably secure within an aperture one or more lines or tubes adjacent to a patient on or near a bed rail or other device. The holding device may be attached for example to a bed rail of a hospital bed and is made of a flexible material.

From WO 88/07386 A1 an intravascular tube assembly is known which facilitates the administration of drugs and fluids to a patient. A plurality of tubes herein are arranged on a generally flat separator which comprises multiple openings into which multiple infusion lines can be inserted. Multiple separators may be displaced from one another along a longitudinal extension direction of the infusion lines.

US 5,005,793 discloses a needle cap holder for holding a cap in a stationary position. The holder may be attached to a pole of a stand for holding an infusion bag, the holder being clamped to the pole. Infusion lines may be guided on the holder.

It is an object of the instant invention to provide a holding device which in an easy and comfortable manner allows for arranging a multiplicity of infusion lines for example on a rack carrying a multiplicity of medical devices, in particular infusion pumps.

The object is achieved with a holding device comprising the features of claim 1.

Accordingly, the tab section of a first of the multiplicity of attachment elements is positioned at a different height, when viewed along the longitudinal direction, than the tab section of a second of the multiplicity of attachment elements.

The holding device comprises a multiplicity of attachment elements, each being defined by a circumferentially extending tab section. The tab section of each attachment element defines a guide opening in which one or several line or cable of a medical device may be received for guiding the line or cable along the longitudinal direction through the guide opening.

In that the tab sections of different attachment elements are positioned at different heights (when viewed along the longitudinal direction) a particularly clear and well-arranged arrangement of multiple lines or cables of medical devices, in particular infusion lines of infusion pumps, on a rack becomes possible. In particular, because different tab sections are arranged at different heights, they can be easily accessed and can be labeled on forward facing front faces such that a user, for example a nurse, in an easy and clear manner can make out which line or cable is arranged in which attachment element.

The tab section of the first of the multiplicity of attachment elements and the tab section of the second of the multiplicity of attachment elements, in one embodiment, are furthermore displaced with respect to each other along a direction perpendicular to the longitudinal direction. The tab sections hence are displaced both along the longitudinal direction and along a direction perpendicular to the longitudinal direction. A first tab section hence may for example be arranged at a higher, rearward position with respect to a second tab section at a lower, forward position.

Beneficially, the tab section of the first of the multiplicity of attachment elements and the tab section of the second of the multiplicity of attachment elements each comprise a front face forming a display area for a label. Hence, on a front face of each tab section a label may be placed for labelling the attachment element associated with the tab section in a unique, identifiable manner, for example by placing numbers, color markers or other identification characters on the front face. Hence, a user may distinguish between the attachment elements in an easy way by looking at their front faces facing the user in a common forward direction.

Each front face, in one embodiment, comprises an upper edge extending transversely to the longitudinal direction. Herein, the upper edge of the tab section of the first of the multiplicity of attachment elements is positioned at a different height than the upper edge of the tab section of the second of the multiplicity of attachment elements such that the tab sections are positioned at different heights. Herein, when viewed along the longitudinal direction, the tab sections may overlap, but are displaced with respect to each other along the longitudinal direction with regard to their upper edges.

In a particular embodiment, each tab section is spring elastic such that the tab section is deformable in a plane perpendicular to the longitudinal direction. For example, the holder may be made of a plastic material. By bending a tab section it, for example, may be allowed to insert one or several line into the guide opening defined by the tab section in an insertion direction perpendicular to the longitudinal direction.

For this, the tab section of each attachment element beneficially comprises an insertion opening discontinuing the tab section in the circumferential direction such that one or several line or cable can be inserted into the guide opening in an insertion direction perpendicular to the longitudinal direction. When viewed along the circumferential direction, each tab section hence is discontinued at one point and hence is circumferentially opened such that the guide opening defined by the tab section is not fully enclosed by the tab section. Through the insertion opening formed by the discontinuation of the tab section one or several line or cable may be inserted transversely to the longitudinal direction into the guide opening.

In one embodiment, the tab sections of the multiplicity of attachment elements are connected to each other by a common connecting element. The connecting element may for example extend along a plane of symmetry of the holding device and may have a blade-like shape.

In such embodiment, the holder has a symmetrical shape and arrangement. In particular, a first portion of the multiplicity of attachment elements in this embodiment may be arranged on a first side of the connecting element and a second portion of the multiplicity of attachment elements may be arranged, symmetrically to the first portion, on a second side of the connecting element.

In a particular example, two attachment elements may be arranged on each side of the connecting element in a symmetrical manner. The attachment elements on each side herein, with their tab sections, are arranged at different heights.

The holder, in another embodiment, is integrally formed in one piece. The holder, for example, may be made of plastics in a single piece including the attachment elements with their tab sections.

To connect the holder to, for example, a rack or to a medical device, the holding device, in one embodiment, comprises a carrying arm that is connected to and extends from a connector of the holder. The carrying arm may, for example, be made of metal and may be shaped as a bent metal strip. At the connector the carrying arm is connected to the holder for example in a positive locking and/or force-locking manner.

A rack for carrying medical devices comprises, in one embodiment, one or multiple holding devices of the type described before. The rack is constituted to hold multiple medical devices, in particular, infusion pumps, the lines or cables of which may be arranged in and guided through the attachment elements of the holding devices arranged on the rack.

Just as well, a medical device, in particular an infusion pump, may comprise one or multiple holding devices of the type described above. A holder of a holding device herein may be connected to a rack or a medical device via the carrying arm, wherein the carrying arm may be fixed to the rack or the medical device for example in a clamping fashion, for example via a clamping screw.

It also is conceivable to connect one or multiple holding devices of the type described above to a pole of a stand to which a rack for carrying a multiplicity of medical devices is attached.

The idea underlying the invention shall subsequently be described in more detail with regard to the embodiments shown in the figures. Herein:
- Fig. 1: shows a schematic view of a rack arranged on a stand for carrying multiple medical devices;
- Fig. 2: shows a view of an embodiment of a holder of a holding device comprising multiple attachment elements for guiding multiple lines or cables of medical devices;
- Fig. 3: shows a view of a holding device in relation to a medical device; and
- Fig. 4: shows a schematic view of a holding device.

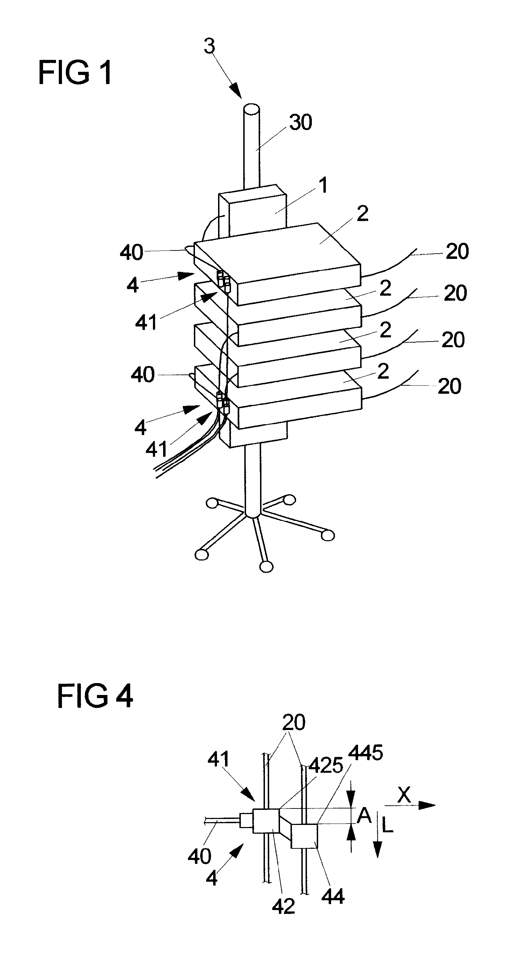

Fig. 1 shows a rack 1 which is constituted to carry multiple medical devices 2, in particular infusion devices such as volumetric infusion pumps or syringe pumps. The rack 1 serves to organize and manage the medical devices 2 for example at a bedside of a patient in a hospital environment. The rack 1 may serve as a communication link providing a communication connection for the medical devices 2 for example to a hospital communication network and/or to each other. The rack 1 furthermore may provide a power supply for the different medical devices 2.

As shown in Fig. 1, the rack 1, shaped as a vertically extending column, is for example arranged on a pole 30 of a stand 3. The stand 3 may be placed at the bedside of a patient in a hospital.

When placing multiple medical devices 2, in particular multiple infusion devices, at the bedside of a patient, it is critical to organize lines or cables of the medical devices 2, in particular infusion lines 20 of infusion devices, such that a risk for errors when administering drugs or other medical fluids to a patient is reduced to a minimum. In this regard it must be made easy for a user, in particular a nurse, to associate infusion lines 20 to corresponding infusion devices 2 and reservoirs containing medical fluids.

To ease the organization of lines or cables 20, in the schematic embodiment of Fig. 1 multiple holding devices 4 are connected to the rack 1, each holding device 4 comprising a holder 41 for receiving multiple lines 20 and a carrying arm 40 for connection the holder 41 to the rack 1.

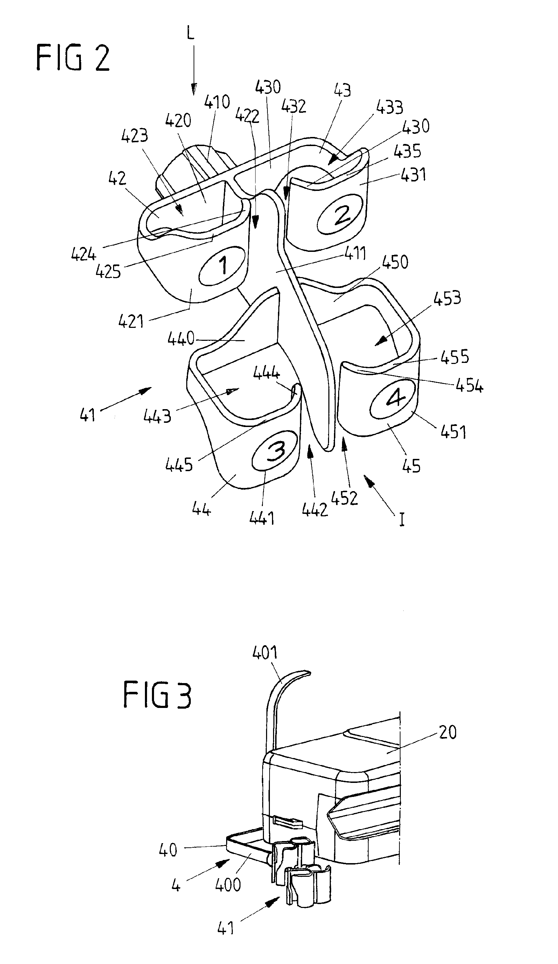

An example of a holder 41 is shown in Fig. 2.

In this embodiment, the holder 41 comprises four attachment elements 42-45, each comprising a tab section 420-450 extending around a guide opening 423-453 for receiving one or several line or cable 20. The different tab sections 420-450 each extend from a central, blade-like connecting element 411 connecting the tab sections 420-450 to each other. The connecting element 411 extends along a plane of symmetry of the holder 41. Two tab sections 420-450 are arranged on each side of the connecting element 411.

The holder 41 is integrally formed in one piece and is made for example of a plastic material. The tab sections 420-450 each extend from the connecting element 411 along a circumferential direction about a longitudinal direction L. Along the longitudinal direction L one or several line or cable 20, when inserted into an attachment element 42-45, extends through the guide opening 423-453 of the attachment element 42-45.

Each tab section 420-450 extending from the connecting element 411 is curved about the longitudinal direction L back towards the connecting element 411 and is placed, with a distal end 424-454, at a distance from the connecting element 411 such that an insertion opening 422-452 is formed between the distal end 424-454 of the tab section 420-450 and the connecting element 411. Through the insertion opening 422-452 one or several line or cable 20 can be inserted into the guide opening 423-453 of an attachment element 42-45 in an insertion direction I perpendicular to the longitudinal direction L.

Each tab section 420-450 may be spring-elastic in a plane perpendicular to the longitudinal direction L (i.e., the plane of the guide opening 423-453). Hence, for inserting one or several line or cable 20 into a guide opening 423-453 through an insertion opening 422-452 the associated tab section 420-450 is elastically bendable such that the insertion opening 422-452 can be widened in the circumferential direction for inserting the line or cable 20.

Each tab section 420-450 has a front face 421-451 facing forward. On the front face 421-451 a labelling, in the embodiment the numbers "1" to "4", is displayed such that a user in an easy way can distinguish between the different attachment elements 42-45.

As visible from Fig. 2 and the schematic drawing of Fig. 4, the attachment elements 42-45 are arranged pair-wise on different heights, when viewed along the longitudinal direction L. Namely, first attachment elements 42, 43 arranged symmetrically to each other are placed at a first height at a higher elevation as compared to second attachment elements 44, 45, also arranged symmetrically to each other, at a second, lower height.

This is indicated in Fig. 4. In particular, upper edges 425-455 of the front faces 421-451 of the attachment elements 42-45 are displaced with respect to each other by a distance A along the longitudinal direction L.

Furthermore, the two pairs of attachment elements 42-45 are also displaced in a direction X perpendicular to the longitudinal direction L. Hence, the attachment elements 42-45 pair-wise are displaced with respect to each other both in the longitudinal direction L and perpendicular to the longitudinal direction L.

Because the rearward attachment elements 42, 43 with their front faces 421, 431 are placed at a higher elevation than the forward attachment elements 44, 45 with their front faces 441, 451, a user in an easy way may make out the labels on the front faces 421-451 of the attachment elements 42-45. It hence becomes easy for a user to arrange lines or cables 20 in an organized manner in multiple holders 41 at a rack 1 carrying multiple medical devices 2.

An arrangement of a holder 41 next to a medical device 2 is illustrated in Fig. 3. The holder 41 is connected via a carrying arm 40 to for example the rack 1, wherein it also is conceivable to connect the holder 41 via the carrying arm 40 to a medical device 2 or to the pole 30 of the stand 3. The carrying arm 40, via a first connecting section 400, is connected to a connector 410 of the holder 41 and via a second connecting section 401 for example to the rack 1.

The carrying arm 40 may be formed for example from a bent metal strip, for example from steel.

The carrying arm 40 herein may be shaped such that it reaches around the medical device 20 for holding the holder 41 in an easily accessible location next to a medical device 2.

As shown in Fig. 1, multiple holding devices 4 with multiple holders 41 may be placed in combination on for example a rack 1. By using multiple holding devices 4 with multiple holders 41 displaced along the longitudinal direction L with respect to each other, a multiplicity of lines or cables 20 may be in an easy way organized on a vertically extending rack 1 carrying multiple medical devices 2.

Herein, multiple lines or cables 20 may also be placed in a single attachment element 42-45. For example, a nurse may choose to place all intravenous (IV) infusion lines in one attachment element 42-45, whereas lines 20 relating to the enteral nutrition are placed in another attachment element 42-45. Hence, each attachment element 42-45 may be constituted to receive multiple lines or cables 20, for example three lines or cables 20. As shown in Fig. 1, multiple holding devices 4 may be used on a rack 1 carrying multiple medical devices, for example twelve or more infusion pumps. For example, a first holding device 4 may be placed at the bottom of the rack 1, whereas a second holding device 4 is placed at a higher location for example in the middle or at the top of the rack 1.

The idea underlying the invention is not limited to the embodiments described above, but may also be implemented in entirely different embodiments.

In particular, a holder may comprise a different number of attachment elements, for example six or eight, wherein it is also conceivable that a holder comprises an odd number of attachment elements.

Further, a holder not necessarily has a symmetrical shape, but may also be shaped asymmetrically.

### List of reference numerals

- 1: Rack
- 2: Medical device
- 20: Infusion line
- 3: Stand
- 30: Pole
- 4: Holding device
- 40: Carrying arm
- 400-401: Connecting section
- 41: Holder
- 410: Connector
- 411: Connecting element
- 42-45: Attachment element
- 420-450: Tab section
- 421-451: Front Face
- 422-452: Insertion opening
- 423-453: Guide opening
- 424-454: Distal end
- 425-455: Upper edge
- A: Distance
- I: Insertion direction
- L: Longitudinal direction
- X: Direction

## Claims

1. A holding device (4) for holding lines (20) or cables of medical devices (2), the holding device (4) comprising a holder (41) having a multiplicity of attachment elements (42-45) integrally connected to each other for receiving a multiplicity of lines (20) or cables, each attachment element (42-45) comprising
- a guide opening (423-453) for receiving one or several line (20) or cable and
- a tab section (420-450) extending along a circumferential direction about a longitudinal direction (L) around the guide opening (423-453),
**characterized in**
**that** the tab section (420-450) of a first of the multiplicity of attachment elements (42-45) is positioned at a different height, when viewed along the longitudinal direction (L), than the tab section (420-450) of a second of the multiplicity of attachment elements (42-45).

2. The holding device (4) according to claim 1, **characterized in that** the tab section (420-450) of the first of the multiplicity of attachment elements (42-45) and the tab section (420-450) of the second of the multiplicity of attachment elements (42-45) are displaced with respect to each other along a direction (X) perpendicular to the longitudinal direction (L).

3. The holding device (4) according to claim 1 or 2, **characterized in that** the tab section (420-450) of the first of the multiplicity of attachment elements (42-45) and the tab section (420-450) of the second of the multiplicity of attachment elements (42-45) each comprise a front face (421-451) forming a display area for a label.

4. The holding device (4) according to claim 3, **characterized in that** each front face (421-451) comprises an upper edge extending transversely to the longitudinal direction (L), the upper edge (421-451) of the tab section (420-450) of the first of the multiplicity of attachment elements (42-45) being positioned at a different height than the upper edge (425-455) of the tab section (420-450) of the second of the multiplicity of attachment elements (42-45).

5. The holding device (4) according to one of the preceding claims, **characterized in that** each tab section (420-450) is spring elastic such that the tab section (420-450) is deformable in a plane perpendicular to the longitudinal direction (L).

6. The holding device (4) according to one of the preceding claims, **characterized in that** the tab section (420-450) of each attachment element (42-45) comprises an insertion opening (422-452) discontinuing the tab section (420-450) in the circumferential direction such that one or several line (20) or cable is insertable into the guide opening (423-453) in an insertion direction (I) perpendicular to the longitudinal direction (L).

7. The holding device (4) according to one of the preceding claims, **characterized in that** the tab sections (420-450) of the multiplicity of attachment elements (42-45) are connected to each other by a connecting element (411).

8. The holding device (4) according to claim 7, **characterized in that** the connecting element (411) extends along a plane of symmetry of the holding device (4), a first portion of the multiplicity of attachment elements (42-45) being arranged on a first side of the connecting element (411) and a second portion of the multiplicity of attachment elements (42-45) being arranged, symmetrically to the first portion, on a second side of the connecting element (411).

9. The holding device (4) according to claim 7 or 8, **characterized in that** the connecting element (411) is shaped as a blade extending in the plane of symmetry.

10. The holding device (4) according to one of the preceding claims, **characterized in that** the holder (41) is integrally formed in one piece.

11. The holding device (4) according to one of the preceding claims, **characterized in that** the holding device (4) comprises a carrying arm (40) for connecting the holder (41) to a rack (1) for carrying medical devices (2) or to a medical device (2), the carrying arm (40) being connected to a connector (410) of the holder (41).

12. A rack (1) for carrying medical devices (2), the rack (1) comprising at least one holding device (4) according to one of the preceding claims.

13. The rack according to claim 12, **characterized in that** the medical devices (2) include infusion pumps, the holding device (4) being constituted to receive a multiplicity of infusion lines (20).
